# EUROPÄISCHE PATENTSCHRIFT

(11) **EP 1 205 542 B1**
(45) Veröffentlichungstag und Bekanntmachung des Hinweises auf die Patenterteilung: **02.08.2006**
(21) Anmeldenummer: 01125294.7
(22) Anmeldetag: 25.10.2001
(51) Int. Cl.: C12N 9/06, C12P 41/00, C12Q 1/68

(54) **L-Aminosäure-Oxidase aus Rhodococcus-Arten**
L-amino acid oxidase from Rhodococcus
L-aminoacide-oxydase de Rhodococcus

(30) Priorität: 09.11.2000 DE 10055512
(43) Veröffentlichungstag der Anmeldung: 15.05.2002
(73) Patentinhaber: Degussa AG, 40474 Düsseldorf (DE)
(72) Erfinder: Geueke, Birgit, 52074 Aachen (DE); Hummel, Werner, Dr., 52445 Titz (DE); Bommarius, Prof. Dr., 30327 Atlanta, GA (US)

(56) Entgegenhaltungen:
- US-A- 5 416 019
- DATABASE EMBL [Online] WHITE O. ET AL.: retrieved from EBI Database accession no. AE001863 XP002189107
- DATABASE EMBL [Online] WHITE O. ET AL.: retrieved from EBI Database accession no. Q9RYN6 XP002189108
- COUDERT M ET AL: "CHARACTERIZATION AND PHYSIOLOGICAL FUNCTION OF A SOLUBLE L AMINO-ACID OXIDASE EC-1.4.3.2 IN CORYNEBACTERIUM" ARCHIVES OF MICROBIOLOGY, Bd. 102, Nr. 2, 1975, Seiten 151-154, XP001055659 ISSN: 0302-8933
- BRUNHUBER N M W ET AL: "CLONING, SEQUENCING, AND EXPRESSION OF RHODOCOCCUS L-PHENYLALANINE DEHYDROGENASE SEQUENCE COMPARISONS TO AMINO-ACID DEHYDROGENASES" JOURNAL OF BIOLOGICAL CHEMISTRY, AMERICAN SOCIETY OF BIOLOGICAL CHEMISTS, BALTIMORE, MD, US, Bd. 269, Nr. 23, 10. Juni 1994 (1994-06-10), Seiten 16203-16211, XP000942066 ISSN: 0021-9258
- MASSAD GEORGE ET AL: "Proteus mirabilis amino acid deaminase: Cloning, nucleotide sequence, and characterization of aad." JOURNAL OF BACTERIOLOGY, Bd. 177, Nr. 20, 1995, Seiten 5878-5883, XP001055638 ISSN: 0021-9193
- ISHII ET AL.: "Genes Encoding Two Isocitrate Dehydrogenase Isozymes of a Psychrophilic Bacterium, Vibrio sp. Strain ABE-1" JOURNAL OF BACTERIOLOGY, Bd. 175, Nr. 21, November 1993 (1993-11), Seiten 6873-6880,

## Beschreibung

Die vorliegende Erfindung betrifft ein Enzym, welches in Rhodococcus-Stämmen vorkommt. Insbesondere bezieht sich die Erfindung auf eine L-Aminosäureoxidase (L-AAO) aus Rhodococcus, speziell Rhodococcus opacus DSM 43250.

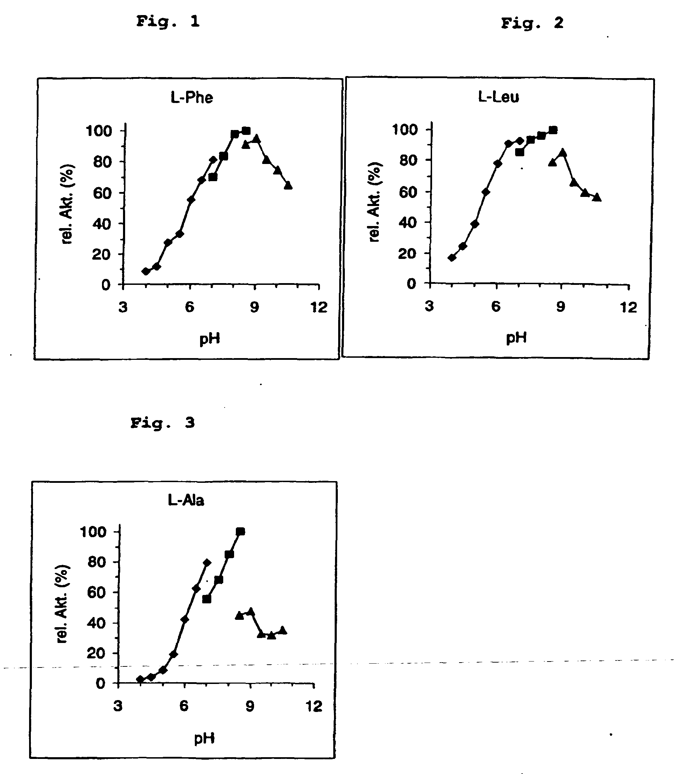

Die am weitesten erforschten unter den bisher bekannten L-Aminosäureoxidasen sind Isolate aus Schlangengift, z.B. aus Crotalus atrox [Torii, S., M. Naito, and T. Tsuruo, Apoxin I, a novel apoptosis-inducing factor with L-amino acid oxidase activity purified from Western diamondback rattlesnake venom. J Biol Chem, 1997. **272**(14): p. 9539-42], Crotalus adamanteus [Raibekas, A.A. and V. Massey, Primary structure of the snake venom L-amino acid oxidase shows high homology with the mouse B cell interleukin 4-induced Fig1 protein. Biochem Biophys Res Commun, 1998. **248**(3): p. 476-8], Calloselasma rhodostoma [Ponnudurai, G., M.C. Chung, and N.H. Tan, Purification and properties of the L-amino acid oxidase from Malayan pit viper (Calloselasma rhodostoma) venom. Arch Biochem Biophys, 1994. **313**(2): p. 373-8], Agkistrodon contortrix laticinctus [Souza, D.H., et al., Isolation and structural characterization of a cytotoxic L-amino acid oxidase from Agkistrodon contortrix laticinctus snake venom: preliminary crystallographic data. Arch Biochem Biophys, 1999. **368**(2): p. 285-90], Bothrops cotiara [Pessatti, M., et al., Screening of Bothrops snake venoms for L-amino acid oxidase activity [published erratum appears in Appl Biochem Biotechnol 1995 Dec;55(3):276] Appl Biochem Biotechnol, 1995. **51-52**: p. 197-210], Lachesis muta muta [Sanchez, E.O. and A. Magalhaes, Purification and partial characterization of an L-amino acid oxidase from bushmaster snake (Surucucu Pico de Jaca) Lachesis muta muta venom. Braz J Med Biol Res, 1991. **24**(3): p. 249-60], Pseudechis australis [Stiles, B.G., F.W. Sexton, and S.A. Weinstein, Antibacterial effects of different snake venoms: purification and characterization of antibacterial proteins from Pseudechis australis (Australian king brown or mulga snake) venom. Toxicon, 1991. **29**(9): p. 1129-41], Ophiophagus hannah [Ahn, M.Y., B.M. Lee, and Y.S. Kim, Characterization and cytotoxicity of L-amino acid oxidase from the venom of king cobra (Ophiophagus hannah). Int J Biochem Cell Biol, 1997. **29**(6): p. 911-9], Naja naja kaouthia [Tan, N.H. and S. Swaminathan, Purification and properties of the L-amino acid oxidase from monocellate cobra (Naja naja kaouthia) venom. Int J Biochem, 1992. **24**(6): p. 967-73]. Weitere Quellen für L-Aminosäureoxidasen sind Algen [Ito, K., K. Hori, and K. Miyazawa, Purification and some properties of L-amino aicd oxidase from Amphiroa crassissima Yendo. Hydrobiologica, 1987. **151/152:** p. 563-569; Piedras, P., et al., Purification and chrarcterization of an L-amino-acid oxidase from Chlamydomonas reinhardtii. Planta, 1992. 188: p. 13-18], das Cyanobakterien Synechococcus [Pistorius, E.K. and H. Voss, Some properties of a basic L-amino-acid oxidase from Anacystis nidulans. Biochim Biophys Acta, 1980. **611**(2): p. 227-40], Pilze [Kusakabe, H., et al., A new antitumor enzyme, L-lysine alpha-oxidase from Trichoderma viride. Purification and enzymological properties. J Biol Chem, 1980. **255**(3): p. 976-81; Le, K.H. and V.R. Villanueva, Purification and characterization of epsilon-N-trimethyllysine L-amino oxidase from Neurospora crassa. Biochim Biophys Acta, 1978. **524**(2): p. 288-96 und Bakterien [Cioaca, C. and A. Ivanof, Bacterial amino acid oxidases. I. L-amino acid oxidase and its distribution in bacteria. Arch Roum Pathol Exp Microbiol, 1974. **33**(3-4): p. 211-22; Gamati, S. and J.H. Luong, Production and purification of L-phenylalanine oxidase from Morganella morganii. Bioseparation, 1991. **2**(3): p. 147-54; Li, Q.S., J.J. Xu, and J.J. Zhong, Production of L-glutamate oxidase and in situ monitoring of oxygen uptake in solid state fermantation of Steptomyces sp. N1. Applied Biochemistry and Biotechnology, 1997. **62**: p. 243-250; Koyama, H., Purification and characterization of a novel L-phenylalanine oxidase (Deaminating and decarboxylating) from Pseudomonas sp. P-501. J Biochem (Tokyo), 1982. **92**(4): p. 1235-40; Brearley, G.M., et al., Purification and partial characterisation of a broad-range L-amino acid oxidase from Bacillus carotarum 2Pfa isolated from soil. Appl. Microbiol. Biotechnol., 1994. **41**(6): p. 670-676; Bouvrette, P. and J.H.T. Luong, Isolation, purification, and further characterization of an L-phenylalanine oxidase from Morganella morganii. Applied Biochemistry and Biotechnology, 1994. **48**: p. 61-74]. Von einigen L-AAOs ist die Gensequenz schon bekannt.

Trotz dieser bekannten Gensequenzen ist es bisher noch nicht gelungen, ein Expressionssystem zu entwickeln, um L-AAO in größeren Mengen herzustellen. Die L-AAO aus Crotalus atrox ist das einzige Enzym, das überhaupt aktiv heterolog exprimiert werden konnte [Torii, S., et al., Molecular cloning and functional analysis of apoxin I, a snake venomderived apoptosis-inducing factor with L-amino acid oxidase activity. Biochemistry, 2000. **39**(12): p. 3197-205]. Die Expression wurde in menschlichen, embryonalen Nierenzellkulturen durchgeführt; das aktive Enzym liegt extrazellulär vor. Die Expression funktionsfähiger L-AAO aus Crotalus atrox in E.coli war nicht erfolgreich. Ein Grund hierfür können die Glykosylierungen oder weitere Modifikationen sein, die im Allgemeinen die Expression funktionsfähiger, eukaryotischer Proteine in mikrobiellen Systemen erschweren oder gar unmöglich machen.

Die Sequenz der prokaryotischen L-AAO aus Synechococcus wurde 1995 erstmals veröffentlicht [Bockholt, R., et al., Partial amino acid sequence of an L-amino acid oxidase from the cyanobacterium Synechococcus PCC6301, cloning and DNA sequence analysis of the aoxA gene. Biochim Biophys Acta, 1995. **1264**(3): p. 289-93], 1998 noch einmal deutlich korrigiert (gi:3341474). Bisher gibt es aber erstaunlicherweise keine Angaben über den wirklichen N-Terminus des aktiven Enzyms. Das mutmaßliche L-AAO-Gen aus Bacillus subtilis ist in der Form, in der es in den Datenbanken zu finden ist, wohl kaum vollständig, da ein Großteil der hochkonservierten FAD-Bindungsstelle fehlt [Vallon, O., New sequence motis in flavoproteins: Evidence for common ancestry and tools to predict structure. Proteins, 2000. **38**(1): p. 95-114]. Auch das angegebene Genprodukt aus Chromobacterium violaceum wurde noch nicht zweifelsfrei nachgewiesen (gi:5802874). Aufgrund dieser verwirrenden und unvollständigen Sequenzinformationen ist auch die Suche nach einem geeignetem Expressionssystem für diese prokayotischen L-AAOs äußerst kompliziert.

Bisher bekannte L-AAOs finden vor allem in der Biosensorik Anwendung [Liu, J. and J. Wang, Remarkable thermostability of bioelectrodes based on enzymes immobilized within hydrophobic semi-solid matrices [In Process Citation]. Biotechnol Appl Biochem, 1999. **30**(Pt 2): p. 177-83; Varadi, M., et al., Determination of the ratio of D- and L-amino acids in brewing by an immobilised amino acid oxidase enzyme reactor coupled to amperometric detection. Biosens Bioelectron, 1999. **14**(3): p. 335-40; Sarkar, P., et al., Screen-printed amperometric biosensors for the rapid measurement of L-and D-amino acids. The Analyst, 1999. **124**: p. 865-870; Lee, Y.C. and M.H. Huh, Development of a biosensor with immobilized L-amino acid oxidase for determination of L-amino acids. Journal of Food Biochemistry, 1998: **1999:** p. 173-185]. In kleinem präparativen Maßstab wurden die Umsetzungen von L-Lysin-Derivaten [Hanson, R.L., et al., Transformation of N epsilon-CBZ-L-lysine to CBZ-L-oxylysine using L- amino acid oxidase from Providencia alcalifaciens and L-2-hydroxy-isocaproate dehydrogenase from Lactobacillus confusus. Appl Microbiol Biotechnol, 1992. **37**(5): p. 599-603], L-Methionin [Takahashi, E., et al., D-methionine preparation from racemic methionines by Proteus vulgaris IAM 12003 with asymmetric degrading activity. Appl. Microbiol. Biotechnol., 1997. **47**(2): p. 173-179] und β-N-Methylamino-L-alanin [Hashmi, M. and M.W. Anders, Enzymatic reaction of beta-N-methylaminoalanine with L-amino acid oxidase. Biochim Biophys Acta, 1991. **1074**(1): p. 36-94] mittels verschiedener L-AAOs untersucht.

Aufgabe der vorliegenden Erfindung war es eine L-AAO zur Verfügung zu stellen, welche sich für den technischen Einsatz in einem Verfahren zur Herstellung von enantiomerenangereicherten Aminosäuren eignet. Insbesondere sollte die L-AAO leicht herstellbar sein und in ausreichender Menge für den genannten Zweck zur Verfügung stehen.

Diese Aufgabe wird gelöst durch eine L-AAO gemäß Anspruch 1. Anspruch 2 ist auf eine bevorzugte L-AAO gerichtet. Anspruch 3 betrifft die für die L-AAOs codierenden Gensequenzen und Ansprüche 4 und 5 betreffen Primer, Vektoren und Mikroorganismen, welche die Gensequenzen bzw. rec-Gensequenzen aufweisen. Ansprüche 6 und 7 betreffen Primer bzw. eine Sonde für die erfindungsgemäßen Gensequenzen und Ansprüche 8 - 10 richten sich auf die Verwendung der erfindungsgemäßen AAOs.

Dadurch, daß man eine L-Aminosäureoxidase (L-AAO) aus Rhodococcus-Arten, welche mit einer Gensonde, die die sequenz der SEQ ID NO:5 aufweist, auffinbar ist, zur Verfügung stellt, besitzt man die Möglichkeit, die gestellte Aufgabe zufriedenstellend zu lösen. Ganz besonders bevorzugt ist dabei der Einsatz einer L-AAO aus Rhodococcus opacus DSM43250 (Seq. 2).Die erfindungsgemäßen Enzyme eignet sich besonders gut für den technischen Einsatz zur Herstellung von enantiomerenangereicherten Aminosäuren. Sie setzen racemische Gemische von Aminosäuren dahingehend um, daß jeweils der L-Anteil des Gemisches in eine Ketosäure umgewandelt wird.

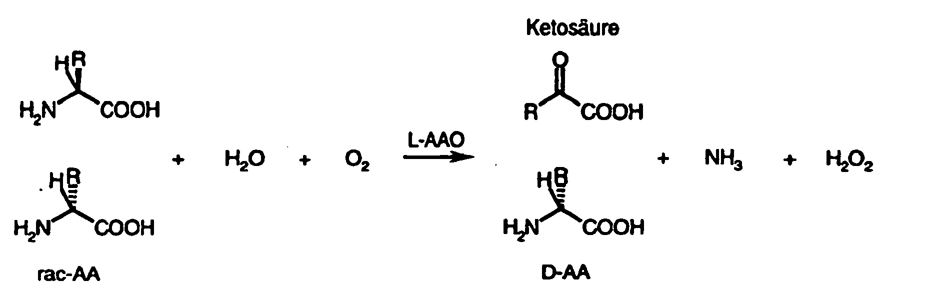

Zurück bleibt der D-Anteil der betrachteten Aminosäure in hoher Enantiomerenreinheit. Die Trennung von Ketosäure und D-Aminosäure kann nach dem Fachmann bekannten Verfahren erfolgen, beispielsweise durch selektive Fällung in saurer wäßriger Lösung oder durch Ionenaustauschchromatographie. Vorteilhaft ist, daß das erfindungsgemäße Enzym ein breites Substratspektrum besitzt, so daß viele natürliche und unnatürliche Aminosäuren umzusetzen sind. Das Gleichgewicht der Reaktion liegt weit auf der rechten Seite der Reaktionsgleichung, so daß die Umsetzung quantitativ verläuft. Das Enzym enthält FAD als Cofaktor. Eine externe Cofaktorregenerierung ist allerdings nicht nötig, da der vorhandene Sauerstoff das FAD spontan oxidiert und somit regeneriert.
Das bei der betrachteten Reaktion entstehende H₂O₂ kann nach dem Fachmann bekannter Manier z.B. mit Katalase zerstört werden.
Ein weiterer nicht minder wichtiger Vorteil dieser L-AAO ist, daß deren Produzenten (Rhodococcus-Arten) einfach kultivierbar sind und z.B. aus Rhodoccocus opacus die L-AAO ohne großen Aktivitätsverlust in nur 3 Chromatographieschritten mit 67% Ausbeute an homogenen Protein erhältlich ist. Weiterhin ist vorteilhaft, daß das Enzym über mehrere Monate ohne Aktivitätsverlust bei 4°C gelagert werden kann.
Ein zusätzlicher positiver Effekt liegt in der Tatsache begründet, daß neben der hoch enantiomerenangereicherten D-Aminosäure auch eine Ketosäure anfällt. Wahlweise bietet sich dieses Verfahren deshalb auch zur Herstellung von sehr reinen Ketosäuren an. Diese werden u.a. in der stickstoffarmen parenteralen Ernährung benötigt. Dafür sind extreme Reinheitsanforderungen an die Ketosäuren zu stellen, die mit chemischen Verfahren schwer oder nur mit extremen Aufwand zu erreichen sind.

Ein weiterer Aspekt der Erfindung beschäftigt sich mit den Gensequenzen, welche für die AAOs aus Rhodococcus codieren. Seq. 1 zeigt die Gensequenz für die L-AAO aus Rhodococcus opacus DSM 43250.

Geschützt sind ebenfalls Plasmide oder Vektoren, welche eine oder mehrere der erfindungsgemäßen Gensequenzen aufweisen. Bevorzugte Plasmide bzw. Vektoren können u. a. die Vektoren der pET-Reihe sein, zusätzlich pKK223-3 und verschiedene pUC-Systeme (mit künstlich eingefügter ribosomaler Bindungsstelle). Ebenfalls bevorzugt sind Plasmide mit Rhamnose oder Arabinose-induzierbaren Promotoren. Allgemein können alle Vektoren mit induzierbaren oder konstitutiven Promotoren und mit natürlichen oder künstlichen ribosomalen Bindungsstellen eingesetzt werden.

Ebenfalls behandelt wird ein Mikroorganismus, der eine oder mehrere Gensequenzen codierend für eine L-AAO aus Rhodococcus-Arten aufweist. Es ist dem Fachmann überlassen, welchen Organismus er zur Expression der L-AAO wählt. Im Prinzip kommen alle dem Fachmann für diesen Zweck bekannten Organismen in Frage. Vorzugsweise wird es sich jedoch um einen prokaroyotischen Organismus handeln, da die L-AAOs ebenfalls aus Bakterienkulturen (Rhodococcus) stammen. Ganz besonders bevorzugt ist in diesem Fall ein E. coli als Expressionssystem, äußerst bevorzugt wird es sich um die Stämme XL 01 blue, JM109, NM522, JM105, RR1, DH5α, TOP10⁻ oder HB101 bzw. BL21(DE3) handeln.

Prinzipiell kommt für die Durchführung der Erfindung jedes dem Fachmann bekannte Plasmid(Vektor)/Wirts-System in Frage, in welches das Gen über eine entsprechende Schnittstelle kloniert bzw. das so entstandene Konstrukt transformiert werden kann. Dem Fachmann sind derartige Systeme geläufig, und er weiß um die Möglichkeit, daß Plasmide mit verschiedenen Wirts-Systemen kombiniert werden können. Eine Übersicht über z.B. das T7-Expressionssystem ist in Studier et al., Methods Enzymol. 1990, 185, 61-69 gegeben. Weitere geeignete Expressionssysteme können in den einschlägig bekannten Prospekten der Firmen Novagen, Promega, New England Biolabs, Clontech sowie Gibco BRL gefunden werden.

Ein weiterer Aspekt der Erfindung beschäftigt sich mit entsprechenden Primern für die erfindungsgemäßen Gensequenzen. Das Ableiten geeigneter Primer geschieht durch den Vergleich von schon bekannten DNA-Sequenzen des gesuchten Gens, oder durch das "Übersetzen" von Aminosäure-Sequenzen in die Ködon-Verwendüng des entsprechenden Organismus (http://www.kazusa.or.jp/codon/cgi-bin/showcodon.cgi?species=Rhodococcus+opacus+[gbbct]). Auch übereinstimmende AS-Sequenzen von Proteinen aus sogenannten Superfamilien sind dabei hilfreich (Firestine et al., Chemistry & Biology 1996, 3, 779-783).
Als vorteilhaft haben sich im Falle R. opacus die für die in Seq. 3 und 4 dargestellten Primer ergeben. Weiterhin sind als bevorzugte Primer die schnittstellentragenden Anfangs- bzw. Endsequenzen des erfindungsgemäßen Gens anzusehen. Geeignete Schnittstellen sind in den oben angesprochenen Prospekten zu finden. Ganz besonders bevorzugt für die Konstruktion von Primersequenzen sind Schnittstellen der Restriktionsenzyme, die nicht in der Gensequenz vorhanden sind, beispielsweise Schnittstellen durch NdeI, HindIII oder EcoRI.

Gegenstand der vorliegenden Erfindung ist ebenfalls eine geeignete Sonde für die erfindungsgemäßen Gensequenzen. Die Herstellung einer Gensonde aus einem Genfragment ist u. a. in Sambrook et al., A laboratory manual, Cold Spring Harbor Laboratory Press, New York (1989) dargestellt und dem Fachmann geläufig. Im Prinzip können deshalb alle dem Fachmann für diesen Zweck als vorteilhaft erscheinende Sonden zur Durchführung der Erfindung herangezogen werden. Im Falle der L-AAO aus Rhodococcus opacus wurde mit den zwei Primern ein 1209 bp großes Fragment des erfindungsgemäßen Gens mittels PCR-Technik amplifiziert. Diese Technik ist ausführlich in Saiki et al., Science (1988), 239, 487-491 gewürdigt und dem Fachmann daher bekannt. Seine Abfolge an Basenpaaren ist in Seq. 5 dargestellt.
Es diente als Teil einer Sonde zum Auffinden des beanspruchten Gens. Für die anderen L-AAOs aus Rhodococcus-Arten ist deshalb entsprechend R. opacus zu verfahren. In diesem speziellen Fall wurde das Sonden-Genfragment zusammen mit der DIG-Markierung der Firma Roche Diagnostics verwendet.

Vorzugsweise werden die erfindungsgemäßen L-AAOs zur Herstellung von D-Aminosäuren und Ketosäuren verwendet. Auch kann das erfindungsgemäße Enzym zur Reinigung von D-Aminosäuren von ihrer optischen Antipode verwendet werden. So können z.B. chemisch hergestellte D-Aminosäuren mit einer geringeren optischen Reinheit in extrem hoch enantiomer angereicherte D-Aminosäuren umgewandelt werden. Besonders bevorzugt erfolgt dieser Prozeß in einem Enzym-Membran-Reaktor (DE 199 10 691.6).

Die genannten Enzyme können in freier Form als homogen aufgereinigte Verbindungen oder als rekombinant hergestellte Enzyme verwendet werden. Weiterhin können die Enzyme auch als Bestandteil eines intakten Gastorganismus eingesetzt werden oder in Verbindung mit der aufgeschlossenen und beliebig hoch aufgereinigten Zellmasse des Wirtsorganismus. Möglich ist ebenfalls die Verwendung der Enzyme in immobilisierter Form (Bhavender P. Sharma, Lorraine F. Bailey and Ralph A. Messing, "Immobilisierte Biomaterialien - Techniken und Anwendungen", Angew. Chem. 1982, 94, 836-852). Vorteilhafterweise erfolgt die Immobilisierung durch Lyophilisation (Dordick et al. J. Am. Chem. Soc. 194, 116, 5009-5010; Okahata et al. Tetrahedron Lett. 1997, 38, 1971-1974; Adlercreutz et al. Biocatalysis 1992, 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-monocetylether) (Goto et al. Biotechnol. Techniques 1997, 11, 375-378). Die Verwendung als CLECs ist ebenfalls denkbar (St Clair et al. Angew Chem Int Ed Engl 2000 Jan, 39(2), 380-383).

Der Begriff enantiomerenangereichert bezeichnet das Vorliegen einer optischen Antipode im Gemisch mit der anderen in > 50%.

Unter Aminosäure wird im Rahmen der Erfindung eine natürliche oder unnatürliche α- bzw. β-Aminosäure verstanden, d.h. daß der am α- bzw. β-C-Atom der α- bzw. β-Aminosäure befindliche Rest leitet sich von einer natürlichen Aminosäure, wie in Beyer-Walter, Lehrbuch der organischen Chemie, S. Hirzel Verlag Stuttgart, 22. Auflage, 1991, S.822f. dargestellt, oder darüberhinaus auch von entsprechenden unnatürlichen Aminosäuren, welche z.B. in DE19903268.8 aufgeführt sind, ab.

Unter Rhodococcus-Arten werden im Rahmen der Erfindung Bakterien verstanden, welche sich gemäß Bergey's Manual of Determinative Bacteriology (Ninth Ed., 1994, Ed.: Hensyl, W.R., Williams and Williams, Baltimore) in Gruppe 22, Subgruppe 1 einordnen lassen.

### Beispiele:

### 1) Screening nach bakteriellen L-Aminosäureoxidase-Bildnern

Eine hohe Anzahl von Bakterienstämmen wurde auf die Fähigkeit zur Bildung von L-Aminosäureoxidasen (L-AAO) untersucht. Die Bakterien wurden in einem für ihr Wachstum geeigneten Medium angezogen und durch Zentrifugation wurde der Medienüberstand von der Bakterienmasse abgetrennt. Es wurde nach extra- und intrazellulärer L-Aminosäureoxidase-Aktivität gesucht. Zum Aufschluß der Zellen wurde mit dem Aufschlußpuffer (50 mM Triethanolamin (TEA), pH 7,0; 1 mM DTT, 1 ml Antischaummittel / 1 L) eine 40%ige Suspension hergestellt (1,5 ml Puffer pro 1 g Zellen). 1,2 g Glasperlen (Ø 0,3 mm) und 700 µL der Zellsuspension wurden in 1,5 mL-Eppendorfreaktionsgefäße gefüllt und diese wurden in gekühlte Halterungen einer Kugelschwingmühle (MM2; Fa. Retsch, Deutschland) gegeben. Der Aufschluss erfolgte durch 10-minütiges Schütteln bei einer Amplitude von 100% und die Abtrennung der Zelltrümmer und der Glasperlen durch Zentrifugation. Die Überstände (Rohextrakte) wurden abpippetiert und zum Nachweis der Enzymaktivität nach folgender Vorschrift eingesetzt:
0,2 M TEA-HCl-Puffer, pH 7,6
0,2 mg/mL o-Dianisidin
25 U/mL Peroxidase aus Meerrettich (POX)
10 mM L-Aminosäure (L-Alanin, L-Phenylalanin, L-Glutaminsäure, L-Prolin, L-Valin und L-Lysin)

Die Messungen wurden in Mikrotiterplatten bei 30°C und 450 nm durchgeführt. Das Gesamtvolumen pro Ansatz betrug 200 µL. Jede der angegebenen L-Aminosäuren wurde einzeln vermessen. Es wurden jeweils 80 µL der Medienüberstände und 10-20 µL der Rohextrakte pro Enzymreaktion (200 µl gesamt) eingesetzt.

Zur Bewertung der einzelnen Stämme (Tabelle 1) wurde entweder die Aktivität in Units (U) angegeben (1 U entspricht der Bildung von 1 µMol H₂O₂ bzw. Farbstoff pro min) oder bei schwacher Aktivität nur das Vorhandensein einer Oxidase-Aktivität registiert.

**Tabelle 1: Organismen mit L-Aminosäure-Aktivität**

| **Stamm** | **DSM No.** | **Aktivität für L-Leucin** | | **Protein** |
|---|---|---|---|---|
| | | **+/-** | **mU/mL.** | **mg/mL** |
| ***Rhodococcus*** opacus, ***vormals*** | 43250 | + | 38 | 20,5 |
| ***Rhodococcus ruber*** | | | | |
| ***Rhodococcus erythropolis*** | 743 | + | | 14,5 |
| ***Rhodoeoccus equi,*** | 43199 | + | | 15,7 |
| ***vormals Nocardia restricta*** | | | | |
| ***Rhodococcus erythropolis,*** | 43188 | + | | 11,7 |
| ***vormals Nocardia calcarea*** | | | | |
| ***Rhodococcus** spec., **vormals*** | 20165 | + | | 11,0 |
| ***Brevibacterium spec.*** | | | | |
| ***Gordona rubropeztinctus,*** | 43248 | + | | 6,9 |
| ***vormals Rhodococcus corallinus*** | | | | |

### 2) Züchtung von Rhodococcus opacus DSM 43250

Zur Enzymgewinnung wurde *Rhodococcus opacus* in folgendem Medium angezogen: 10 g Malzextrakt; 4 g Hefeextrakt; 4 g Glucose; 1 L dest. H₂O.

Der pH-Wert der Lösung wurde auf pH 7,2 eingestellt, dann wurde für 20 min bei 121°C und 1 bar Überdruck sterilisiert. Der Organismus wurde aerob gezüchtet. Im 10 L-Maßstab wurde der Organismus bei 28°C im Fermenter (Infors, Bottmingen, Schweiz) bei einem pO₂ über 40% ohne pH-Regulierung kultiviert. Nach 23 h wurde bei einer OD₆₀₀ von 10,7 durch Zentrifugation 148 g feuchte Zellmasse geerntet. Die Zellmasse kann bei -20°C tiefgefroren gelagert werden, ohne dass nach mehreren Monaten ein Aktivitätsverlust zu erkennen ist.

### 3) Enzymisolierung

### a) Rohextrakt-Gewinnung

Die Zellen wurden durch Naßvermahlung mit Glasperlen aufgeschlossen. Dazu wurde die Bakterienfeuchtmasse (15 g) in 50 mM TEA-HCl-Puffer (pH 7,0) suspendiert, so dass die Konzentration der Zellfeuchtmasse 25%ig war. Die Zellinhaltsstoffe wurden aus der eisgekühlten Suspension durch einen mechanischen Aufschluss mit Hilfe einer Glasperlenmühle (SCP-Desintegrator, Innomed-Konsult AB, Schweden) freigesetzt. Der Mahlbehälter wurde dazu mit 100 mL Glasperlen (∅ 0,3 mm) und der Zellsuspension gefüllt. Der Aufschluss wurde bei einer Rührwellendrehzahl von 3000 Upm durchgeführt. Der Kühlmantel wurde vor Beginn des Laufes mit Eiswasser befüllt.

15 g Bakterienfeuchtmasse ergaben 45 mL Rohextrakt mit einer Volumenaktivität von 0,035 U/mL und einem Proteingehalt von 9,4 mg/mL. Hieraus lässt sich errechnen, dass aus 10 L Fermenteransatz ca. 150 Units L-Aminosäureoxidase gewonnen werden können.

### b) Chromatographische Aufreinigung des Enzyms

Das Enzym kann durch Ionenaustausch-Chromatographie, hydrophobe Interaktionschromatographie und Chromatographie mit Hydroxylapatit als Säulenmaterial gereinigt werden. Der Rohextrakt wurde auf eine mit MarcoPrep High Q Support Material (Biorad, Hercules, USA) gepackte Säule (h 5,5 x ∅5 cm) mit einer Flußgeschwindigkeit von 2 mL/min aufgepumpt. Der Ionenaustauscher war zuvor mit einem Puffer equilibriert worden, der 50 mM TEA-HCl, pH 7,0 enthielt.
Die Säule wurde anschliessend mit diesem Puffer nachgewaschen, dann wurde das Enyzm mit einem stufenweisen Gradienten von 0 bis 0,5 M NaCl in Startpuffer eluiert. Zu Beginn der Elution wurde die Flußrate auf 4 mL/min erhöht. Die L-Aminosäureoxidase eluierte mit ca. 0,15 M NaCl. Die aktiven Fraktionen wurden vereinigt und mit 3,5 M (NH₄)₂SO₄₋Lösung versetzt, so daß die Endkonzentration an Ammoniumsulfat 0,75 M (NH₄)₂SO₄ betrug. Diese Lösung wurde auf eine mit Phenylsepharose 6FF Material (Amersham Pharmacia Biotech, Uppsala, Schweden) gepackte Säule (h 4,2 x ∅2,6 cm) mit einer Flußgeschwindigkeit von 2 mL/min aufgetragen. Die Säule war zuvor mit einem 50 mM TEA-HCl-Puffer, pH 7,0, der 0,75 M (NH₄)₂SO₄ enthielt, equilibriert worden. Auch diese Säule wurde nach Aufgabe der Probe mit Startpuffer nachgewaschen. Das Enzym eluierte innerhalb des stufenweisen Gradienten von 0,75 bis 0 M (NH₄)₂SO₄ in Startpuffer bei 0,5 M (NH₄)₂SO₄. Die aktiven Fraktionen wurden vereinigt und in einer 50 mL fassenden Ultrafiltrationeinheit (Amicon, Boston, USA) ein Pufferwechsel durchgeführt. Dazu wurde eine Ultrafiltrationsmembran YM10 mit einem Durchmesser von 43 mm und einer Ausschlussgrenze von 10 kDa benutzt (Amicon, Boston, USA). Das Enzym lag nach dem Pufferwechsel in 10 mM Kaliumphosphat-Puffer, pH 6,5, mit 250 mM NaCl gelöst vor. Eine mit keramischem MacroPrep Hydroxylapatit, Type I (Biorad, Hercules, USA) gefüllte Säule (h8,3 x ∅1,6 cm) wurde mit dem selben Puffer equilibriert. Nach dem Aufpumpen der Probe mit einer Flußrate von 1,5 mL/min wurde die Säule mit Startpuffer gewaschen und das Enzym in einem stufenweisen Gradienten von 10 mM bis 200 mM Kaliumphosphat-Puffer, pH 6,5, eluiert. Die L-Aminosäureoxidase eluierte bei einer Kaliumphosphatkonzentration von 50 mM. Der Elutionspuffer enthielt ebenfalls 250 mM NaCl und war auf pH 6,5 eingestellt. Die aktiven Fraktionen wurden erneut durch Ultrafiltration in 50 mM Glycin-NaOH, pH 8,6 umgepuffert und bei 4°C aufbewahrt. Das Ergebnis der Aufreinigungsschritte ist in Tabelle 2 zusammengefasst. Das aufgereinigte Enzym hat eine spezifische Aktivität von 4,6 U/mg mit L-Alanin als Substrat.

**Tabelle 2: Reinigungstabelle der L-Aminosäureoxidase aus Rhodococcus opacus. Enzymtest: 0,2 M TEA-HCl, pH 7,6; 10 mM L-Ala; 25 U/mL POX; 0,2 mg/mL o-Dianisidin; L-AAO; 30°C, 450 nm.**

| | **Volumen (mL)** | **Gesamtprotein (mg)** | **Gesamtaktivität (U)** | **Spez. Akt. (U/mg)** | **Reinigungsfaktor (-fach)** | **Ausbeute (%)** |
|---|---|---|---|---|---|---|
| Ftohextraxt | 25 | 262 | 9,6 | 0,03 | 1 | 100 |
| MacroQ-Pool | 110 | 17 | 12,6 | 0 , 7 4 | 23 | 130 |
| Phenylsepharose -Pool | 35 | 2,9 | 8,2 | 2,82 | 88 | 85 |
| Hydroxylapatit-Pool | 26 | 1,4 | 6,5 | 4,61 | 144 | 67 |

### 4) Biochemische Kenndaten der L-Aminosäure-Oxidase aus Rhodococcus opacus

### a) pH-Abhängigkeit der Oxidation

Die Reaktionsgeschwindigkeit der Oxidation von L-Aminosäuren zu Ketosäuren in Gegenwart der L-Aminosäureoxidase wurde in Abhängigkeit vom pH-Wert in der Reaktionslösung untersucht. Der Testansatz (200 µL) hatte folgende Zusammensetzung: 10 mM L-Aminosäure, 25 U/mL Peroxidase aus Meerrettich, 80 mM Puffer verschiedener pH-Werte und Zusammensetzungen, 0,2 mg/mL o-Dianisidin, Enzym in limiterender Menge. Das Enzym hat für L-Alanin, L-Phenylalanin und L-Leucin ein pH-Optimum bei pH 8,5 in 80 mM TEA-HCl-Puffer (**Fig. 1-3:** pH-Optimum der L-Aminosäureoxidase für die Substrate L-Phenylalanin (a), L-Leucin (b) und L-Alanin (c) in verschiedenen Puffern)

### b) Lagerstabilität der L-Aminosäureoxidase bei 4°C

Unter sterilen Bedingungen ist das aufgereinigte Enzym bei einer Proteinkonzentration von 0,7 mg/mL in 50 mM Glycin-NaOH-Puffer, pH 8,6, für mehrere Monate ohne Aktivitätsverlust bei 4°C lagerbar.

### c) Lagerstabilität der L-Aminosäureoxidase in Abhängigkeit vom pH-Wert

L-Aminosäureoxidase wurde in 0,2 M Puffer unterschiedlicher Zusammensetzung bei einer Proteinkonzentration von 24 µg/mL bei Raumtemperatur inkubiert. Danach wurde die Restaktivität unter Verwendung von 0,2 M TEA-Puffer, pH 7,6 und 10 mM L-Ala bestimmt. Dabei zeigte sich eine gute pH-Stabilität im Bereich von pH 10 bis 10,5. Nach 100 Stunden in Glycin-NaOH-Puffer waren bei pH 10 und 10,5 noch über 90% der Aktivität nachweisbar.

### d) Bestimmung der kinetischen Konstanten Kₘ, Kᵢ und vₘₐₓ für verschiedene L-Aminosäuren

Zur Bestimmung der kinetischen Konstanten wurde die Reaktionsgeschwindigkeit der Oxidation bei verschiedenen Konzentrationen an Aminosäure und für verschiedene L-Aminosäuren unter folgenden Bedingungen untersucht (Tests in in Mikrotiterplatten):

0,2 M TEA-HCl-Puffer, pH 7,6; 0,2 mg/mL o-Dianisidin; 25 U/mL POX; L-Aminosäureoxidase in limitierenden Mengen; variierende Konzentrationen der verschiedenen L-Aminosäuren Aktivitätsmessung wurde photometrisch bei 450 nm und 30 °C durchgeführt.

Die gefundenen kinetischen Konstanten vₘₐₓ, Kₘ und Kᵢ sind in Tabelle 3 zusammengefaßt.

**Tabelle 3: Kinetische Daten (Kₘ-, Kᵢ- und vₘₐₓ-Werte) der enzymatischen Oxidation verschiedener L-Aminosäuren**

| **Substrat** | **Vmax (U/mg)** | **Km (mM)** | **Ki (mM)** |
|---|---|---|---|
| **L-Ala** | 4,27 | 0,274 | - |
| **L-Arg** | 8,12 | 0,070 | 5,42 |
| **L-Asn** | 5,37 | 0,028 | - |
| **L-Cit** | 5,47 | 0,026 | 20,6 |
| **L-Gln** | 5, 06 | 0,085 | - |
| **L-Glu** | 2,32 | 0,411 | - |
| **L-Ile** | 2,84 | 5,11 | - |
| **L-Leu** | 6,45 | 0,028 | 5,69 |
| **L-Lys** | 3,56 | 0,015 | 63,7 |
| **L-Met** | 6,43 | 0,039 | 6,84 |
| **L-Orn** | 7,48 | 0,034 | 12,5 |
| **L-Phe** | 5,64 | 0,019 | 5,29 |
| **L-Ser** | 4,36 | 1,36 | 192 |
| **L-Val** | 1,93 | 3,73 | - |

### e) Substratspektrum

Die Umsetzung einer großen Anzahl natürlicher und nichtnatürlicher Aminosäuren wurde untersucht. Folgender Testansatz (1 mL) wurde dazu verwendet: 10 mM enantiomerenreine Aminosäure (oder 20 mM D,L-Aminosäure); 0,2 M TEA-HCl-Puffer, pH 7,6; 0,2 mg/mL o-Dianisidin; 10 U/mL POX; L-Aminosäureoxidaselösung in limiterienden Mengen. Die Aktivitätsmessungen wurden photometrisch bei 436 nm und 30 °C durchgeführt.

Die Stereospezifität der L-Aminosäureoxidase wurde für mehrere Substrate (z. B. Alanin, Asparagin, Glutamin, Phenylalanin, Methionin, Leucin, Ornithin, Lysin, Histidin) gemessen, indem sowohl die D- und die L-Form als auch das Racemat eingesetzt wurden. D-Aminosäuren wurden dabei in keinem Fall umgesetzt. Die Aktivitäten mit den Racematen waren für alle Substrate ebenso hoch wie mit den jeweiligen reinen L-Aminosäuren. Dies zeigt, daß die L-Aminosäureoxidase nicht durch D-Aminosäuren inhibiert wird. Bei der Bestimmung des Substratspektrums kann somit auch die D,L-Form eingesetzt werden.

Die gemessenen Aktivitäten wurden als relative Werte in Bezug auf die Aktivität mit 10 mM L-Alanin angegeben (Tabelle 4). Schwache Aktivitäten wurden durch ein + in der Tabelle markiert.

### Tabelle 4: Substratspezifität der L-Aminosäureoxiclase

Die mit * gekennzeichneten Aminosäuren konnten aufgrund ihrer schlechten Wasserlöslichkeit nur in geringeren Konzentrationen eingesetzt werden. Hier wurden pro Testansatz 100 µL des Überstandes einer gesättigten L-Aminosäurelösung verwendet.

Die mit gekennzeichneten Aminosäuren wurden in racemischer Form eingesetzt.

| | **L-Aminosäure** | **Rel. Aktivität (%)** |
|---|---|---|
| Aliphatische Aminosäuren | Alanin | 100 |
| | Leucin | 68 |
| | Valin | 32 |
| | Isoleucin | 45 |
| | Norleucin^{#} | 73 |
| | Neopentylglycin | 46 |
| | 2-Amino-5,5-dimethylhexansäure | 44 |
| Aromatische Aminosäuren | Phenylalanin | 53 |
| | Tyrosin* | 54 |
| | Histidin | 36 |
| | Tryptophan | 12 |
| | Homophenylalanin*^{#} | 50 |
| | Phenylglycin* | 5 |
| | 2-Chlorphenylalanin | 24 |
| | 4-Chlorphenylalanin | 9 |
| | 4-Fluorphenylalanin^{#} | 25 |
| | 4-Nitrophenylalanin* | 30 |
| | 4-Aminophenylalanin | 16 |
| | 3-(1-Naphtyl)alanin* | 109 |
| | 3-(1-Pyridyl)alanin^{#} | 24 |
| Basische Aminosäuren | Arginin | 70 |
| | Lysin | 55 |
| | Glutamin | 52 |
| | Asparagin | 97 |
| | Ornithin | 117 |
| | Citrullin | 74 |
| | Nε-Acetyllysin | 46 |
| | 2,3-Diaminopropionsäure | 71 |
| | 2,4-Diaminobuttersäure | 100 |
| Cyclische Aminosäuren | Homocysteinthiolacton | 23 |
| | 2-Amino-4-butyrolacton | 66 |
| S/O haltige Aminosäuren | Serin | 72 |
| | Methionin | 72 |
| | Cystin* | 104 |
| | Cysteinsäure | 34 |
| | O-Methylserin^{#} | 99 |
| | β-Phenylserin^{#} | 8 |
| Saure Aminosäuren | Glutaminsäure | 52 |
| | Asparaginsäure | 11 |
| β-Aminosäuren | β-Homophenylalanin | + |
| | β-Homoalanin | + |

### 5) Proteinchemische Kenndaten der L-Aminosäure-Oxidase aus Rhodococcus opacus

### a) Bestimmung des Molekulargewichts der L-Aminosäureoxidase und Ermittlung der Untereinheiten

Das Molekulargewicht der nativen Enzyms wurde durch Gelfiltration mit Superdex 200 PG (Amersham Pharmacia, Uppsala, Schweden) ermittelt. Die an ein FPLC-System gekoppelte Säule (h61,5 x ∅1,6 cm) wurde mit einer Durchflußrate von 1 mL/min betrieben, wobei als Probe 0,95 mL des hochaufgereinigten Enzyms mit einer Proteinkonzentration von 0,7 mg/mL aufgetragen wurde. Als Eichproteine wurden Ribonuclease A, Chymotrypsinogen, Ovalbumin, Rinderalbumin, Aldolase, Catalase, Ferritin, Thyroglobulin verwendet. Mit Hilfe dieser Eichproteine wurde mit dieser Methode ein Molekulargewicht von 99 kDa für die native Oxidase ermittelt.

Durch Gelelektrophorese in einem 10%igen Acrylamidgel in Gegenwart von Natriumdodecylsulfat (SDS) konnte die Größe und Anzahl der Untereinheiten des Enzyms bestimmt werden. Das Molekulargewicht der Untereinheit beträgt danach 57,7 kDa. Für die Eichung wurden Phosphorylase B, Rinderserumalbumin, Aldolase, Triosephosphatisomerase, Trypsininhibitor, Lysozym verwendet. Die L-Aminosäureoxidase besteht demnach aus 2 identischen Untereinheiten. Die aus der Sequenz errechnete Größe (siehe Beispiel 5c) lag bei 53348 Da.

### b) Bestimmung des isoelektischen Punktes

Der isoelektrische Punkt der L-Aminosäureoxidase wurde mittels isoelektrischer Fokussierung mit Trägerampholyten auf Agarose in einem pH-Bereich von 4-6 ermittelt. Der pI liegt bei pH 4,8. Als Markerproteine wurden Carboanhydrase, β-Lactoglobulin, Trypsininhibitor, Glucoseoxidase eingesetzt.

### c) Bestimmung der Protein- und Gensequenz

Die hochaufgereinigte L-Aminosäureoxidase wurde nach der Vorschrift von Kyse und Anderson aus einem SDS-haltigen, 10%igen Acrylamidgel auf eine Polyvinylidenfluoridmembran transferiert, dann wurde das Protein durch automatisierten Edman-Abbau N-terminal sequenziert. Verwendet wurde ein Automated Sequencer 4774 (Applied Biosystems) mit online HPLC 120 A.

Zur Gewinnung weiterer, interner Sequenzen wurden 200 µg des homogen aufgereinigten Enzyms enzymatisch mittels LysC-Protease (aus *Lysobacter enzymogenes*) gespalten. Die Peptide wurden über präparative RP-HPLC (Säule: Nucleosil 100-5 C8 ET 250/4 (Macherey-Nagel, Düren, Deutschland), Laufmittel A: 0,1% Trifluoressigsäure, Laufmittel B: 0,085% Trifluoressigsäure + 84% Acteonitril, Detektor: 215 nm) getrennt und ebenfalls N-terminal sequenziert. Ausgehend von diesen Sequenzinformationen konnten ca. 80% des L-Aminosäureoxidasegens über PCR amplifiziert werden. Dieses Fragment wurde digoxigeninmarkiert und als Sonde in einer Hybridisierung nach Southern eingesetzt. Genomische DNA aus *Rhodococcus opacus*, die mit dem Restriktionsenzym SmaI verdaut worden war, ergab nach Auftrennung über ein Agarosegel und Transfer auf eine Nylonmembran in der Nachweisreaktion ein Signal bei 3,4 kb. Klonierung von SmaI-geschnittener DNA dieser Größe in einen pUC18-Vektor mit anschließender Transformation in *E.coli* XL 1 blue ergaben einen Klon, der das gewünschte Fragment trug. Die Gensequenz und die daraus abgeleitete Proteinsequenz können dem Anhang entnommen werden.

### 6) Verwendung der L-Aminosäure-Oxidase aus Rhodococcus opacus zur Herstellung von D-Aminosäuren

Eine 7 mM Lösung von D,L-Phenylalanin wurde mit L-Aminosäureoxidase (35 µg/mL homogenes Enzym) in 80 mM TEA-Puffer, pH 7,6 unter Zugabe von 2600 U/mL Katalase umgesetzt. Das Gesamtvolumen des Ansatzes betrug 1 mL. In regelmäßigen Abständen wurden Proben entnommen (50 µL) und mit Hilfe der HPLC auf den Gehalt von L- und D-Phenylalanin analysiert. Direkt nach der Entnahme wurden die Proben für 3 min auf 95°C erhitzt und danach wurde das ausgefallene Protein abzentrifugiert. 20 µL des verdünnten Überstandes wurden in 180 µL 100 mM Natrium-Borat-Puffer, pH 10,4 gegeben und mit 20 µL eines Derivatisierungsreagens (260 mM Isobuturyl-L-Cystein (IBLC) und 170 mM o-Phthaldialdehyd (OPA) in 100 mM Natrium-Borat-Puffer, pH 10,4) versetzt.

Parallel hierzu wurde ein Ansatz mit einer 7 mM Lösung von D,L-Leucin hergestellt und genau wie oben beschrieben verfahren.

Trennbedingungen der HPLC:
Säule: RP-18, 250x4 mm, 5 µm, Kromasil (Fa. Phenomenex, Aschaffenburg, Deutschland)
Detektion: Fluoreszenz, Ex. 340 nm, Em. 440 nm
Laufmittel A: 23 mM Natriumacetat-Puffer; pH 6,5 mit Essigsäure eingestellt
Laufmittel B: Acetonitril:Wasser (10:1,5)

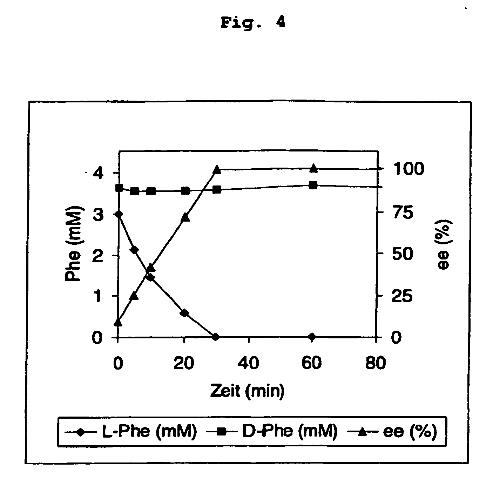

**Fig. 4** zeigt, daß im Verlauf der Reaktion L-Phenylalanin von anfänglich 3,5 mM auf <0,01 mM abnimmt, während die Konzentration an D-Phenylalanin konstant bleibt. Der ee-Wert für D-Phenylalanin beträgt nach 30 min Reaktionszeit ≥99,2%, nach 180 min Reaktionszeit ≥99,5%.

L-Leucin wird während der ersten 20 min fast vollständig umgesetzt. Die Konzentration an D-Leucin bleibt dabei konstant. Nach 20 min Reaktionszeit wurde ein ee-Wert von ≥99,2% gemessen.

## Patentansprüche

1. L-Aminosäureoxidase (L-AAO) erhältlich aus Rhodococcus-Arten, auffindbar mit einer Gensonde, die die Sequenz der SEQ ID NO: 5 aufweist.

2. L-AAO gemäß Anspruch 1 erhältlich aus Rhodococcus opacus DSM43250.

3. Isolierte Gensequenzen codierend für die L-AAO gemäß Anspruch 1 oder 2.

4. Plasmide oder Vektoren aufweisend eine oder mehrere Gensequenzen nach Anspruch 3.

5. Prokaiyantischer gastorganismus aufweisend eine oder mehrere Gensequenzen nach Anspruch 3.

6. Primer für eine Gensequenz nach Anspruch 3.

7. Sonde für eine Gensequenz nach Anspruch 3.

8. Verwendung der L-AAO gemäß Anspruch 1 oder 2 zur Herstellung von D-Aminosäuren bzw. Ketosäuren.

9. Verwendung der L-AAO gemäß Anspruch 1 oder 2 zur Reinigung von D-Aminosäuren von ihrer optischen Antipode.

10. Verwendung nach Anspruch 8 oder 9,
**dadurch gekennzeichnet, daß**
man die Reaktion in einem Enzym-Membran-Reaktor durchführt.

## Claims

1. L-Amino acid oxidase (L-AAO) which can be obtained from Rhodococcus species and which can be found using a gene probe which exhibits the SEQ ID NO:5 sequence.

2. L-AAO according to Claim 1 which can be obtained from Rhodococcus opacus DSM43250.

3. Isolated gene sequences which encode the L-AAO according to Claim 1 or 2.

4. Plasmids or vectors which exhibit one or more gene sequences according to Claim 3.

5. Prokaryotic host organism which exhibits one or more gene sequences according to Claim 3.

6. Primers for a gene sequence according to Claim 3.

7. Probe for a gene sequence according to Claim 3.

8. Use of the L-AAO according to Claim 1 or 2 for preparing D-amino acids or keto acids.

9. Use of the L-AAO according to Claim 1 or 2 for purifying D-amino acids from their optical antipodes.

10. Use according to Claim 8 or 9, **characterized in that** the reaction is carried out in an enzyme membrane reactor.

## Revendications

1. L-aminoacide oxydase (L-AAO) provenant d'espèces appartenant au genre *Rhodococcus,* décelable au moyen d'une sonde génique ayant la séquence de SEQ ID n° 5.

2. L-AAO selon la revendication 1, pouvant être obtenue à partir de *Rhodococcus opacus* DSM43250.

3. Séquences de gènes isolées codant pour la L-AAO selon la revendication 1 ou 2.

4. Plasmides ou vecteurs comportant une ou plusieurs séquences de gènes selon la revendication 3.

5. Organisme hôte procaryote comportant une ou plusieurs séquences de gènes selon la revendication 3.

6. Amorce pour une séquence de gène selon la revendication 3.

7. Sonde pour une séquence de gène selon la revendication 3.

8. Utilisation de la L-AAO selon la revendication 1 ou 2, pour la production de D-aminoacides ou de cétoacides.

9. Utilisation de la L-AAO selon la revendication 1 ou 2, pour la purification de D-aminoacides par séparation d'avec leurs inverses optiques.

10. Utilisation selon la revendication 8 ou 9, **caractérisée en ce qu'**on effectue la réaction dans un réacteur à membrane-enzyme.


### SEQUENZPROTOKOLL

<110> Degussa-HÜls Aktiengesellschaft
<120> L-Aminosäure-Oxidase aus Rhodococcus-Arten
<130> 000607 AM
<140>
   <141>
<160> 5
<170> PatentIn Ver. 2.1
<210> 1
   <211> 1470
   <212> DNA
   <213> Rhodococcus opacus
<220>
   <221> CDS
   <222> (1)..(1470)
<400> 1
   gcc ggc gac ctg atc ggc aag gtg aag ggc agc cat tcc gtg gtc gtg    48
   Ala Gly Asp Leu Ile Gly Lys Val Lys Gly Ser His Ser Val Val Val
     1               5                  10                  15
   ctc ggc ggc ggc ccc gcc ggt ctg tgt tcg gca ttc gaa ctg cag aag    96
   Leu Gly Gly Gly Pro Ala Gly Leu Cys Ser Ala Phe Glu Leu Gln Lys
                20                  25                  30
   gcc ggg tac aag gtg acg gtc ctc gag gcc cgc acc cgg ccc ggt ggc   144
   Ala Gly Tyr Lys Val Thr Val Leu Glu Ala Arg Thr Arg Pro Gly Gly
            35                  40                  45
   cgc gtc tgg acc gca cgg ggc ggc agc gag gag acc gac ctg agc ggc   192
   Arg Val Trp Thr Ala Arg Gly Gly Ser Glu Glu Thr Asp Leu Ser Gly
        50                  55                  60
   gag acg cag aag tgc acg ttc tcg gag ggc cac ttc tac aac gtc ggc   240
   Glu Thr Gln Lys Cys Thr Phe Ser Glu Gly His Phe Tyr Asn Val Gly
    65                  70                  75                  80
   gcc acc cgc atc ccg cag agc cac atc acg ctc gac tac tgc cgc gaa   288
   Ala Thr Arg Ile Pro Gln Ser His Ile Thr Leu Asp Tyr Cys Arg Glu
                    85                  90                  95
   ctc ggc gtc gag atc cag gga ttc gga aac cag aac gcc aac acg ttc   336
   Leu Gly Val Glu Ile Gln Gly Phe Gly Asn Gln Asn Ala Asn Thr Phe
               100                 105                 110
   gtg aac tac cag agc gac acg tcg ctg tct ggc cag tcc gtc acc tac   384
   Val Asn Tyr Gln Ser Asp Thr Ser Leu Ser Gly Gln Ser Val Thr Tyr
           115                 120                 125
   cgg gcc gcg aag gcc gac acg ttc ggc tac atg tcg gaa ctg ctg aag   432
   Arg Ala Ala Lys Ala Asp Thr Phe Gly Tyr Met Ser Glu Leu Leu Lys
           130                 135                 140
   aag gcc acc gat cag ggt gcc ctg gat cag gta ctg agc cgg gag gac      480
   Lys Ala Thr Asp Gln Gly Ala Leu Asp Gln Val Leu Ser Arg Glu Asp
   145                 150                 155                 160
   aag gat gcg ctg tcg gag ttc ctc agc gac ttc ggt gac ctg tcc gac      528
   Lys Asp Ala Leu Ser Glu Phe Leu Ser Asp Phe Gly Asp Leu Ser Asp
                   165                 170                 175
   gac ggc cgc tac ctc gga tcc tcg cgt cgc ggt tac gat tcc gag ccc      576
   Asp Gly Arg Tyr Leu Gly Ser Ser Arg Arg Gly Tyr Asp Ser Glu Pro
               180                 185                 190
   gga gcc ggc ctg aac ttc ggc acc gag aag aag ccg ttc gcg atg cag      624
   Gly Ala Gly Leu Asn Phe Gly Thr Glu Lys Lys Pro Phe Ala Met Gln
           195                 200                 205
   gaa gtg atc cgc agc ggc atc ggc cgc aac ttc agc ttc gac ttc ggc      672
   Glu Val Ile Arg Ser Gly Ile Gly Arg Asn Phe Ser Phe Asp Phe Gly
       210                 215                 220
   tac gac cag gcg atg atg atg ttc acc ccg gtc ggc ggc atg gac cgg      720
   Tyr Asp Gln Ala Met Met Met Phe Thr Pro Val Gly Gly Met Asp Arg
   225                 230                 235                 240
   atc tac tac gcg ttc cag gac agg atc ggc acc gac aac atc gtc ttc      768
   Ile Tyr Tyr Ala Phe Gln Asp Arg Ile Gly Thr Asp Asn Ile Val Phe
                   245                 250                 255
   ggc gcc gag gtg acg tcg atg aag aac gtg tcc gag ggc gtc acc gtc      816
   Gly Ala Glu Val Thr Ser Met Lys Asn Val Ser Glu Gly Val Thr Val
               260                 265                 270
   gaa tac acc gcc ggc ggc tcg aag aag tcg atc acc gcc gac tac gcg      864
   Glu Tyr Thr Ala Gly Gly Ser Lys Lys Ser Ile Thr Ala Asp Tyr Ala
           275                 280                 285
   atc tgc acg atc ccg ccg cac ctc gtc gga cga ctg cag aac aat ctg      912
   Ile Cys Thr Ile Pro Pro His Leu Val Gly Arg Leu Gln Asn Asn Leu
       290                 295                 300
   ccc ggc gac gtg ctc acc gcg ctg aag gcg gcc aag ccg tcg tcg tcc      960
   Pro Gly Asp Val Leu Thr Ala Leu Lys Ala Ala Lys Pro Ser Ser Ser
   305                 310                 315                 320
   gga aag ctc ggc atc gag tac tcg cgc cgg tgg tgg gag acg gag gac     1008
   Gly Lys Leu Gly Ile Glu Tyr Ser Arg Arg Trp Trp Glu Thr Glu Asp
                   325                 330                 335
   cgc atc tac ggc ggc gcg tcc aac acc gac aag gac atc tcg cag atc     1056
   Arg Ile Tyr Gly Gly Ala Ser Asn Thr Asp Lys Asp Ile Ser Gln Ile
               340                 345                 350
   atg ttc ccg tac gac cac tac aac tcc gat cgc ggt gtg gtc gtc gcc     1104
   Met Phe Pro Tyr Asp His Tyr Asn Ser Asp Arg Gly Val Val Val Ala
           355                 360                 365
   tac tac agc agc ggc aag cgt cag gag gcg ttc gag tcc ctc acg cac     1152
   Tyr Tyr Ser Ser Gly Lys Arg Gln Glu Ala Phe Glu Ser Leu Thr His
       370                 375                 380
   cgc cag cgg ctc gcc aag gcg atc gcg gag ggc tcg gag atc cac ggc     1200
   Arg Gln Arg Leu Ala Lys Ala Ile Ala Glu Gly Ser Glu Ile His Gly
   385                 390                 395                 400
   gag aag tac acc cgc gac atc tcg tcg tcg ttc tcg ggc agc tgg cgg     1248
   Glu Lys Tyr Thr Arg Asp Ile Ser Ser Ser Phe Ser Gly Ser Trp Arg
                   405                 410                 415
   cgc acc aag tac tcc gag agt gcc tgg gcc aac tgg gcg ggc agt ggc     1296
   Arg Thr Lys Tyr Ser Glu Ser Ala Trp Ala Asn Trp Ala Gly Ser Gly
               420                 425                 430
   gga tcg cac ggc ggg gcg gcc act ccc gag tac gag aag ctg ctc gaa     1344
   Gly Ser His Gly Gly Ala Ala Thr Pro Glu Tyr Glu Lys Leu Leu Glu
           435                 440                 445
   ccc gtc gac aag atc tat ttc gcc ggc gac cac ctg tcc aac gcc atc     1392
   Pro Val Asp Lys Ile Tyr Phe Ala Gly Asp His Leu Ser Asn Ala Ile
       450                 455                 460
   gcc tgg cag cac ggc gcc ctg acg tcc gcc cgc gac gtc gtc acc cac     1440
   Ala Trp Gln His Gly Ala Leu Thr Ser Ala Arg Asp Val Val Thr His
   465                 470                 475                 480
   atc cac gag cgc gtg gcc cag gaa gcc tga                             1470
   Ile His Glu Arg Val Ala Gln Glu Ala
                   485                 490
<210> 2
   <211> 489
   <212> PRT
   <213> Rhodococcus opacus
<400> 2
   Ala Gly Asp Leu Ile Gly Lys Val Lys Gly Ser His Ser Val Val Val
   1               5                   10                  15
   Leu Gly Gly Gly Pro Ala Gly Leu Cys Ser Ala Phe Glu Leu Gln Lys
               20                  25                  30
   Ala Gly Tyr Lys Val Thr Val Leu Glu Ala Arg Thr Arg Pro Gly Gly
           35                  40                  45
   Arg Val Trp Thr Ala Arg Gly Gly Ser Glu Glu Thr Asp Leu Ser Gly
       50                  55                  60
   Glu Thr Gln Lys Cys Thr Phe Ser Glu Gly His Phe Tyr Asn Val Gly
   65                  70                  75                  80
   Ala Thr Arg Ile Pro Gln Ser His Ile Thr Leu Asp Tyr Cys Arg Glu
                   85                  90                  95
   Leu Gly Val Glu Ile Gln Gly Phe Gly Asn Gln Asn Ala Asn Thr Phe
               100                 105                 110
   Val Asn Tyr Gln Ser Asp Thr Ser Leu Ser Gly Gln Ser Val Thr Tyr
           115                 120                 125
   Arg Ala Ala Lys Ala Asp Thr Phe Gly Tyr Met Ser Glu Leu Leu Lys
       130                 135                 140
   Lys Ala Thr Asp Gln Gly Ala Leu Asp Gln Val Leu Ser Arg Glu Asp
   145                 150                 155                 160
   Lys Asp Ala Leu Ser Glu Phe Leu Ser Asp Phe Gly Asp Leu Ser Asp
                       165                 170                 175
   Asp Gly Arg Tyr Leu Gly Ser Ser Arg Arg Gly Tyr Asp Ser Glu Pro
                   180                 185                 190
   Gly Ala Gly Leu Asn Phe Gly Thr Glu Lys Lys Pro Phe Ala Met Gln
               195                 200                 205
   Glu Val Ile Arg Ser Gly Ile Gly Arg Asn Phe Ser Phe Asp Phe Gly
           210                 215                 220
   Tyr Asp Gln Ala Met Met Met Phe Thr Pro Val Gly Gly Met Asp Arg
   225                 230                 235                 240
   Ile Tyr Tyr Ala Phe Gln Asp Arg Ile Gly Thr Asp Asn Ile Val Phe
                   245                 250                 255
   Gly Ala Glu Val Thr Ser Met Lys Asn Val Ser Glu Gly Val Thr Val
               260                 265                 270
   Glu Tyr Thr Ala Gly Gly Ser Lys Lys Ser Ile Thr Ala Asp Tyr Ala
           275                 280                 285
   Ile Cys Thr Ile Pro Pro His Leu Val Gly Arg Leu Gln Asn Asn Leu
       290                 295                 300
   Pro Gly Asp Val Leu Thr Ala Leu Lys Ala Ala Lys Pro Ser Ser Ser
   305                 310                 315                 320
   Gly Lys Leu Gly Ile Glu Tyr Ser Arg Arg Trp Trp Glu Thr Glu Asp
                   325                 330                 335
   Arg Ile Tyr Gly Gly Ala Ser Asn Thr Asp Lys Asp Ile Ser Gln Ile
               340                 345                 350
   Met Phe Pro Tyr Asp His Tyr Asn Ser Asp Arg Gly Val Val Val Ala
           355                 360                 365
   Tyr Tyr Ser Ser Gly Lys Arg Gln Glu Ala Phe Glu Ser Leu Thr His
       370                 375                 380
   Arg Gln Arg Leu Ala Lys Ala Ile Ala Glu Gly Ser Glu Ile His Gly
   385                 390                 395                 400
   Glu Lys Tyr Thr Arg Asp Ile Ser Ser Ser Phe Ser Gly Ser Trp Arg
                   405                 410                 415
   Arg Thr Lys Tyr Ser Glu Ser Ala Trp Ala Asn Trp Ala Gly Ser Gly
               420                 425                 430
   Gly Ser His Gly Gly Ala Ala Thr Pro Glu Tyr Glu Lys Leu Leu Glu
           435                 440                 445
   Pro Val Asp Lys Ile Tyr Phe Ala Gly Asp His Leu Ser Asn Ala Ile
       450                 455                 460
   Ala Trp Gln His Gly Ala Leu Thr Ser Ala Arg Asp Val Val Thr His
   465                 470                 475                 480
   Ile His Glu Arg Val Ala Gln Glu Ala
                   485
<210> 3
   <211> 39
   <212> DNA
   <213> Künstliche Sequenz
<220>
   <223> Beschreibung der künstlichen Sequenz:Primer1
<400> 3
   atggccggcg acctcatcgg caaggtcaag ggctcgcac 39
<210> 4
   <211> 39
   <212> DNA
   <213> Künstliche Sequenz
<220>
   <223> Beschreibung der künstlichen Sequenz:Primer2
<400> 4
   cttggcgagg cgctggcggt gggtgagcga ctctaaggc 39
<210> 5
   <211> 1209
   <212> DNA
   <213> Künstliche Sequenz
<220>
   <223> Beschreibung der künstlichen Sequenz:Sonde
<400> 5
   atggccggcg acctcatcgg caaggtcaag ggctcgcacg ccggcgacct gatcggcaag 60
   gtgaagggca gccattccgt ggtcgtgctc ggcggcggcc ccgccggtct gtgttcggca 120
   ttcgaactgc agaaggccgg gtacaaggtg acggtcctcg aggcccgcac ccggcccggt 180
   ggccgcgtct ggaccgcacg gggcggcagc gaggagaccg acctgagcgg cgagacgcag 240
   aagtgcacgt tctcggaggg ccacttctac aacgtcggcg ccacccgcat cccgcagagc 300
   cacatcacgc tcgactactg ccgcgaactc ggcgtcgaga tccagggatt cggaaaccag 360
   aacgccaaca cgttcgtgaa ctaccagagc gacacgtcgc tgtctggcca gtccgtcacc 420
   taccgggccg cgaaggccga cacgttcggc tacatgtcgg aactgctgaa gaaggccacc 480
   gatcagggtg ccctggatca ggtactgagc cgggaggaca aggatgcgct gtcggagttc 540
   ctcagcgact tcggtgacct gtccgacgac ggccgctacc tcggatcctc gcgtcgcggt 600
   tacgattccg agcccggagc cggcctgaac ttcggcaccg agaagaagcc gttcgcgatg 660
   caggaagtga tccgcagcgg catcggccgc aacttcagct tcgacttcgg ctacgaccag 720
   gcgatgatga tgttcacccc ggtcggcggc atggaccgga tctactacgc gttccaggac 780
   aggatcggca ccgacaacat cgtcttcggc gccgaggtga cgtcgatgaa gaacgtgtcc 840
   gagggcgtca ccgtcgaata caccgccggc ggctcgaaga agtcgatcac cgccgactac 900
   gcgatctgca cgatcccgcc gcacctcgtc ggacgactgc agaacaatct gcccggcgac 960
   gtgctcaccg cgctgaaggc ggccaagccg tcgtcgtccg gaaagctcgg catcgagtac 1020
   tcgcgccggt ggtgggagac ggaggaccgc atctacggcg gcgcgtccaa caccgacaag 1080
   gacatctcgc agatcatgtt cccgtacgac cactacaact ccgatcgcgg tgtggtcgtc 1140
   gcctactaca gcagcggcaa gcgtcaggag cttggcgagg cgctggcggt gggtgagcga 1200
   ctctaaggc                                                         1209